(19) Europäisches Patentamt
European Patent Office
Office européen des brevets

(11) Veröffentlichungsnummer : **0 363 761 B1**

(12) # EUROPÄISCHE PATENTSCHRIFT

(45) Veröffentlichungstag der Patentschrift :
**03.03.93 Patentblatt 93/09**

(51) Int. Cl.$^5$ : **C07D 209/48**

(21) Anmeldenummer : **89118115.8**

(22) Anmeldetag : **29.09.89**

(54) **Aromatische Etherimide.**

(30) Priorität : **12.10.88 DE 3834660**

(43) Veröffentlichungstag der Anmeldung :
**18.04.90 Patentblatt 90/16**

(45) Bekanntmachung des Hinweises auf die Patenterteilung :
**03.03.93 Patentblatt 93/09**

(84) Benannte Vertragsstaaten :
**BE DE ES FR GB IT NL**

(56) Entgegenhaltungen :
**EP-A- 0 249 262**
**EP-A- 0 254 994**
**US-A- 3 833 544**
**US-A- 3 944 583**
**US-A- 3 968 083**

(73) Patentinhaber : **BAYER AG**
**W-5090 Leverkusen 1 Bayerwerk (DE)**

(72) Erfinder : **Reuter, Knud, Dr.**
**Scheiblerstrasse 99**
**W-4150 Krefeld (DE)**
Erfinder : **Freitag, Dieter, Dr.**
**Hasenheide 10**
**W-4150 Krefeld (DE)**
Erfinder : **Weymanns, Günther, Dr.**
**Karl-Arnold-Strasse 4**
**W-5090 Leverkusen 1 (DE)**
Erfinder : **Dhein, Rolf, Dr.**
**Deswatinesstrasse 30**
**W-4150 Krefeld (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue aromatische Etherimide, ein Verfahren zu ihrer Herstellung sowie die Verwendung der neuen aromatischen Etherimide zur Herstellung von Kunststoffen und Kunststoffmischungen, die zu Formkörpern, Filmen, Folien und Filamenten weiterverarbeitet werden können.

US-P 3 944 583 beschreibt aromatische Etherimide mit einer Bisphenolfluorenon-Bindungsgruppe. Diese Etherimide können verwendet werden zur Herstellung von Polymeren mit einer guten Wärmeformbeständigkeit.

Gegenstand der Erfindung sind neue aromatische Etherimide der Formeln (I), (II) und (III)

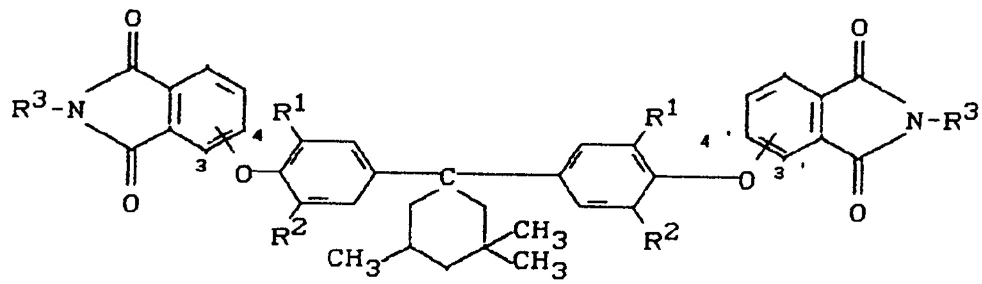

(I)

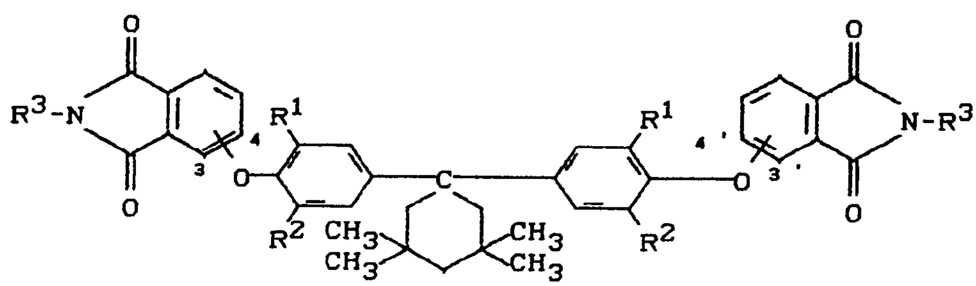

(II) und

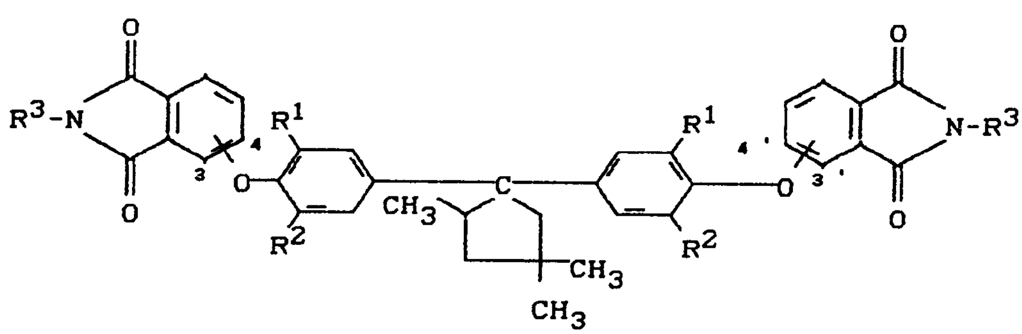

(III),

worin

$R^1$ und $R^2$ unabhängig voneinander Wasserstoff, Halogen, $C_1$-$C_8$-Alkyl, $C_5$-$C_6$-Cycloalkyl, $C_6$-$C_{10}$-Aryl oder $C_7$-$C_{12}$-Aralkyl bedeuten und

$R^3$ für $C_1$-$C_4$-Alkyl, $C_5$-$C_6$-Cycloalkyl oder $C_6$-$C_{10}$-Aryl steht.

Als Halogene der oben angegebenen Formeln kommen beispielsweise in Frage Fluor, Chlor und Brom, insbesondere Brom und Chlor, als Alkylreste der Methyl-, Ethyl-, Propyl- und Butylrest, bevorzugt der Methylrest, als Cycloalkylreste der Cyclopentyl- und der Cyclohexylrest, bevorzugt der Cyclohexylrest, als Arylreste

der Phenyl- und Naphthylrest, bevorzugt der Phenylrest, und als Aralkylreste der Benzyl- und der Cumylrest, bevorzugt der Cumylrest.

Bevorzugt sind aromatische Etherimide der Formeln (I), (II) und (III), in denen die Reste $R^1$ und $R^2$ unabhängig voneinander Wasserstoff oder Methyl bedeuten und $R^3$ für Methyl oder Phenyl steht. Besonders bevorzugt sind Verbindungen der Formeln (I), (II) und (III), in denen $R^1$ und $R^2$ Wasserstoff bedeuten und $R^3$ für Methyl steht.

Ein weiterer Gegenstand der vorliegenden Erfindung ist ein Verfahren zur Herstellung von aromatischen Etherimiden der Formeln (I), (II) und (III)

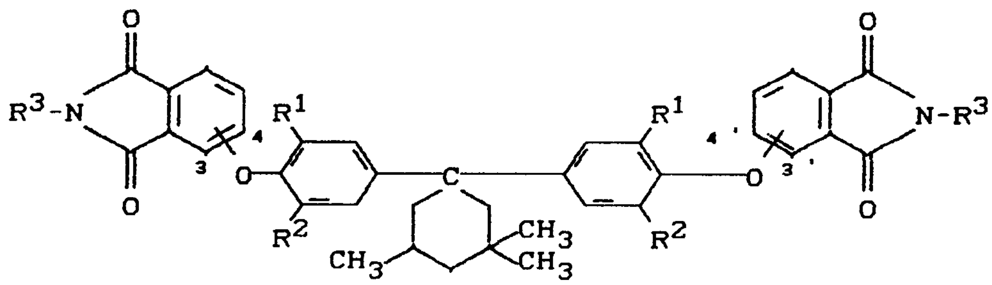

(I)

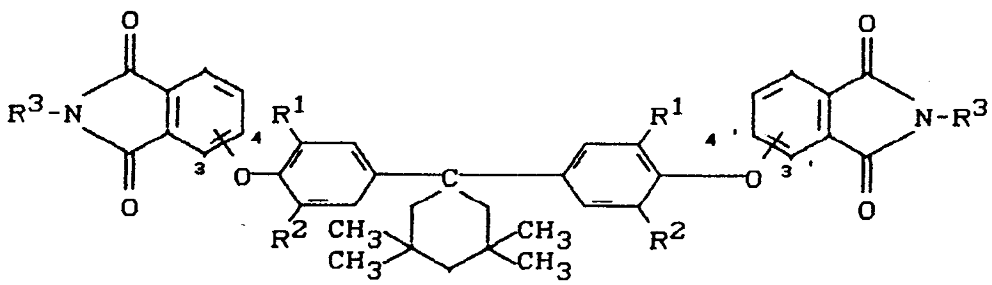

(II) und

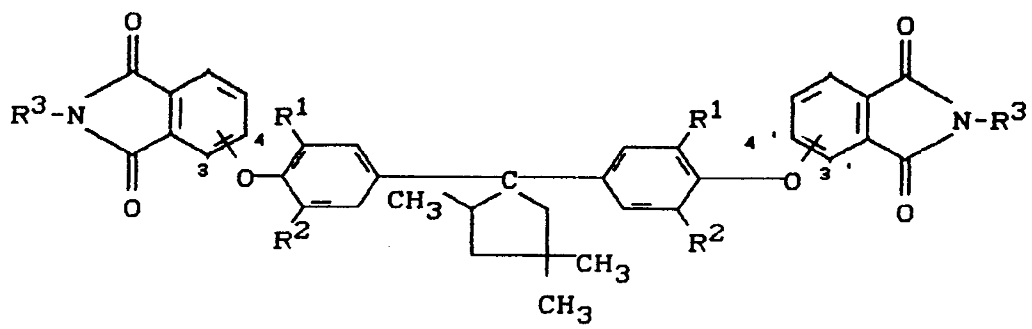

(III),

worin

$R^1$ und $R^2$ unabhängig voneinander Wasserstoff, Halogen, $C_1$-$C_8$-Alkyl, $C_5$-$C_6$-Cycloalkyl, $C_6$-$C_{10}$-Aryl oder $C_7$-$C_{12}$-Aralkyl bedeuten und

$R^3$ für $C_1$-$C_4$-Alkyl, $C_5$-$C_6$-Cycloalkyl oder $C_6$-$C_{10}$-Aryl steht,

dadurch gekennzeichnet, daß man Verbindungen der Formeln

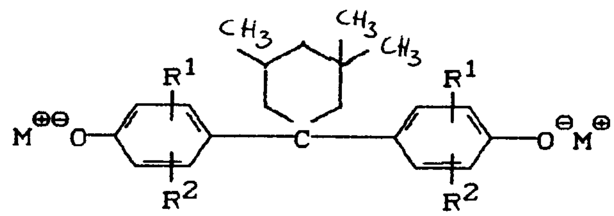

(IV),

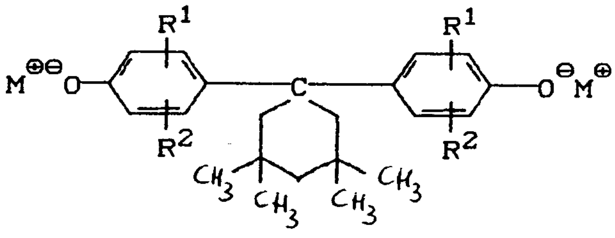

und

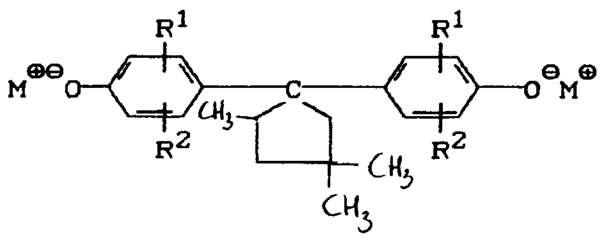

,

worin

$R^1$ und $R^2$ die obengenannte Bedeutung haben und

M für ein Alkalimetall, insbesondere für Lithium, Natrium oder Kalium, steht,

mit Verbindungen der Formel (V)

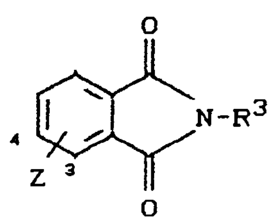

(V),

worin

$R^3$ die obengennante Bedeutung besitzt und

Z für Fluor, Chlor, Brom, Iod oder die Nitrogruppe steht, bevorzugt Nitro, Fluor oder Chlor, ganz besonders bevorzugt Nitro bedeutet,

bei Temperaturen von 20 bis 180° C, bevorzugt 50 bis 150° C, in Gegenwart eines dipolaren, aprotischen Lösungsmittels umsetzt.

Die Verbindungen der Formel (IV) werden in Mengen von ca. 2 bis 3 Mol, bevorzugt 2 bis 2,5 Mol, bezogen auf 1 Mol der Verbindung der Formel (VI) eingesetzt.

Als dipolare, aprotische Lösungsmittel werden bevorzugt genannt: Acetonitril, Diethylenglykoldimethylether, N,N-Dimethylacetamid, N-Methylpyrrolidon, N-Methylcaprolactam, N,N-Dimethylformamid und/oder Dimethylsulfoxid, Besonders bevorzugt ist Dimethylsulfoxid. Es ist auch möglich, einen Teil, vorzugsweise bis zu ca, 50 Gew,-%, der dipolaren, aprotischen Lösungsmittel zu ersetzen durch unpolare Lösungsmittel, wie Toluol, Xylol, Mesitylen, Chlorbenzol, Cyclohexan und/oder Petrolether.

Die Menge des eingesetzten Lösungsmittels kann in weiten Bereichen schwanken. Im allgemeinen werden ca, 0,5 bis 50, bevorzugt 2 bis 20 Gew.-Teile Lösungsmittel, bezogen auf die Gesamtmenge an eingesetzter Verbindung der Formel (IV) und (V), verwendet, Verwiesen wird in diesem Zusammenhang auf die US-PS

3.873.593, in der nähere Erläuterungen zu dem oben beschriebenen Verfahren zu entnehmen sind.

Die eingesetzten Verbindungen der Formel (V) sind bekannt und beispielsweise beschrieben in Flitsch, Chem. Ber. 94, S. 2498 (1961), Williams & Donahue, J. Org. Chem. 42 (21), S. 3414 ff, (1977), Markezich & Zamek, J. Org. Chem. 42 (21), S. 3431 (1977) oder können hergestellt werden nach dem in der US-PS 4 005 102 beschriebenen Verfahren.

Die aromatischen Dihydroxyverbindungen der Formel (IV) können hergestellt werden durch Kondensation in an sich bekannter Weise der entsprechenden Phenole mit den entsprechenden Ketonen in Gegenwart von sauren Katalysatoren sowie gegebenenfalls weiteren Cokatalysatoren. Verwiesen wird in diesem Zusammenhang auf die deutsche Patentanmeldung P 38 32 396.6 und auf Schnell, Chemistry and Physics of Polycarbonates, Interscience Publishers, New York, 1964.

Als in das erfindungsgemäße Verfahren einzusetzende Verbindungen der Formel (IV) kommt bevorzugt in Frage:

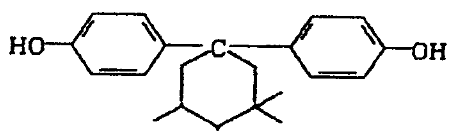

Als Verbindungen der Formel (V) können z.B. eingesetzt werden:

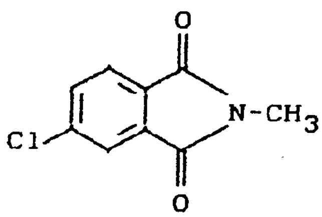 , 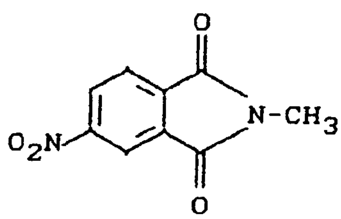 ,

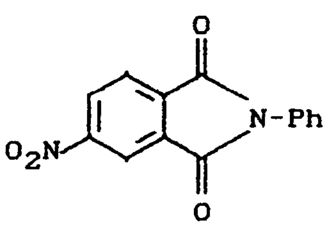 , 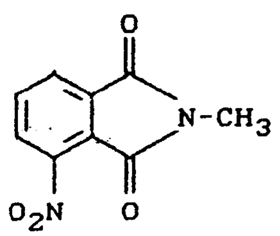 ,

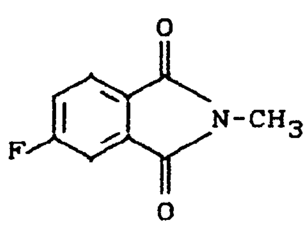

bevorzugt

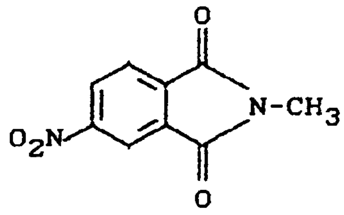

und

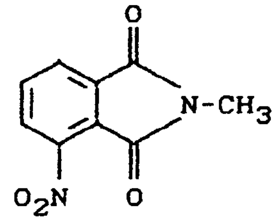

,

besonders bevorzugt

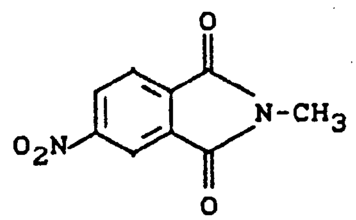

.

Ein weiterer Gegenstand der vorliegenden Erfindung ist die Verwendung der neuen aromatischen Etherimide zur Herstellung von Polymeren, insbesondere von Duroplasten und Thermoplasten. Die aus den neuen aromatischen Ethern hergestellten Polymeren können wiederum verwendet werden für die Herstellung von Formkörpern, Filmen, Filamenten und Folien.

Beispielsweise können die neuen aromatischen Etherimide eingesetzt werden für die Herstellung von Polyetherimiden, Ethertetracarbonsäuren oder Ethertetracarbonsäuredianhydride.

Die neuen aromatischen Etherimide oder geeignete Folgeprodukte (Ethercarbonsäuren oder Ethercarbonsäureanhydride) werden nach üblicher Weise mit den für die einzelnen Kunststoffe in Frage kommenden anderen Comonomeren polykondensiert. Die Herstellung der oben angeführten Polymere kann dabei nach literaturbekannten Verfahren (siehe beispielsweise US-Patentschriften 3 833 544, 3 847 870, 3 850 885, 3 875 116, 3 905 942, 3 998 840, 4 011 198, 4 073 773, 4 324 884, 4 324 882, 4 324 885, 4 324 883), durchgeführt werden.

Es ist dabei auch möglich, die erfindungsgemäßen Etherimide oder deren zur Kunststoffherstellung verwendeten Folgeprodukte (Ethercarbonsäuren, Ethercarbonsäureanhydride) im Gemisch mit anderen geeigneten Bausteinen zum Polykondensat umzusetzen, z.B. Pyromellithsäure(di-anhydrid). Außerdem ist es möglich, bekannte Etherimide und deren Folgeprodukte, wie in den obengenannten US-Patentschriften beschrieben, anteilig zur Polykondensation einzusetzen.

Selbstverständlich ist es auch möglich, die aus den neuen aromatischen Etherimiden hergestellten Polymeren untereinander oder mit anderen bekannten Polymeren, wie Polycarbonaten, Polyestercarbonaten, Polyestern, Polyimiden, Polyamiden, Polyetherketonen, Polyethersulfonen und/oder aromatischen Polyethern in üblicher Weise und in üblichen Mischungsverhältnissen abzumischen.

Die aus den neuen aromatischen Etherimiden hergestellten Polymeren besitzen gegenüber vergleichbaren Polymeren auf Basis von bekannten Hydroxydiphenylcycloalkanen eine besonders gute Wärmeformbeständigkeit.

Beispiele

Beispiel 1

46,6 g des Bisphenols

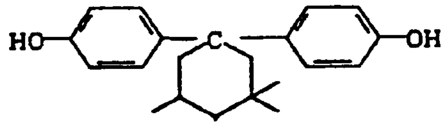

(0,15 M), 26,7 g 45 %ige NaOH (0,3 M), 200 ml Dimethylsulfoxid und 130 ml Toluol werden unter $N_2$ in einer Rührapparatur mit Wasserabscheider zum Rückfluß erhitzt, bis kein Wasser mehr abgeschieden wird. Danach wird der Wasserabscheider durch eine Soxhlet-Apparatur, die mit Molekularsieb 4 Å beschickt ist, ersetzt und die Mischung eine weitere Stunde zum Rückfluß erhitzt. Anschließend wird die Soxhlet-Apparatur durch einen absteigenden Kühler ersetzt und bis zu einer Innentemperatur von 145°C eindestilliert, wodurch in der Hauptsache Toluol entfernt wird.

Danach werden 68,3 g 4-Nitro-N-methylphthalimid (0,33 M) zugegeben und das Reaktionsgemisch 6 h unter $N_2$ auf 60°C erhitzt. Nach Ende der Reaktionszeit und Abkühlung auf Raumtemperatur werden durch Abnutschen 64,1 g Produkt isoliert, die je einmal mit Methanol und $H_2O$ gewaschen werden. Aus der Mutterlauge werden nach Stehen über Nacht weitere 13,3 g Produkt isoliert und mit Methanol und $H_2O$ gewaschen; Gesamtausbeute 77,4 g = 82 % der Theorie. Das Produkt (beide Fraktionen) hat einen Schmelzpunkt von 201 bis 203°C und wird durch NMR-Spektroskopie und Elementaranalyse (C 74,5 %, H 5,96 %, N 4,67 %; Theorie C 74,5 %, H 5,77 %, N 4,46 %) als Verbindung folgender Struktur identifiziert:

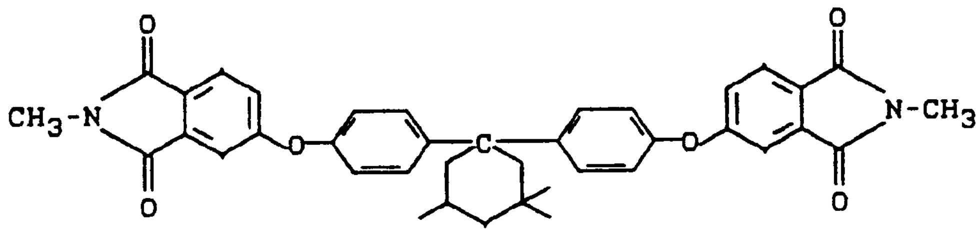

Beispiel 2

Analog Beispiel 1 wurden 23,3 g des Bisphenols

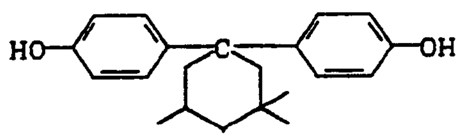

(0,075 M), 13,35 g 45 %ige NaOH (0,15 M), 100 ml Dimethylsulfoxid, 65 ml Toluol und 34,15 g 3-Nitro-N-methylphthalimid (0,165 M) umgesetzt. Nach Aufarbeitung entsprechend Beispiel 1 wurden 39,0 g (83 % der Theorie) der Verbindung

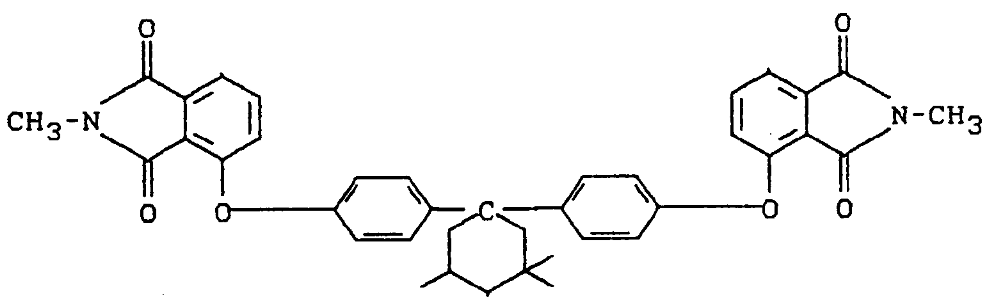

vom Schmelzpunkt 244-245°C isoliert und die Struktur durch NMR-Spektroskopie bestätigt; Elementaranalyse:

| | C | H | N |
|---|---|---|---|
| berechnet | 74,5 | 5,77 | 4,46 % |
| gefunden | 74,3 | 5,78 | 4,47 % |

Beispiel 3

282,9 g des Bisimids

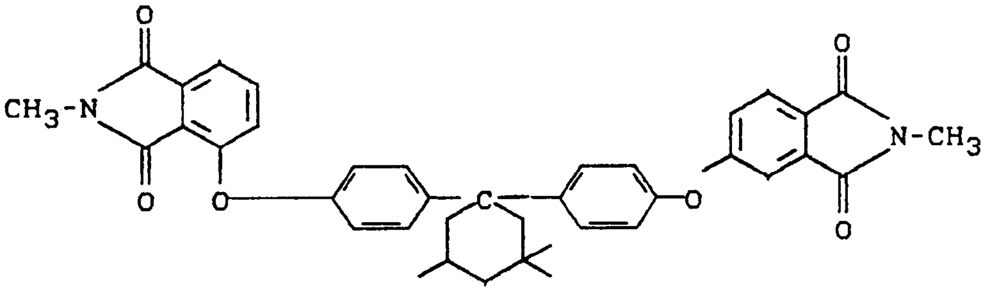

(entsprechend Beispiel 1 hergestellt),
272,3 g 45 %ige NaOH, 200 ml Ethanol und 450 ml $H_2O$ werden 4 1/2 Std. am Rückfluß erhitzt. Danach wird die Lösung mit $H_2SO_4$ auf einen pH-Wert von 1 eingestellt und
die Fällung mit $H_2O$ gewaschen. Das Produkt wird durch sein NMR-Spektrum und die Elementaranalyse als die Tetracarbonsäure

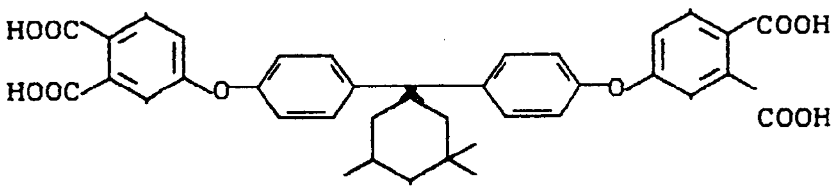

identifiziert.

Elementaranalyse:

| | C | H |
|---|---|---|
| berechnet | 69,6 | 5,37 % |
| gefunden | 70,3 | 5,20 % |

Beispiel 4

107,6 g der Tetracarbonsäure aus Beispiel 3 werden in 357 g Essigsäureanhydrid 1 h zum Rückfluß erhitzt und anschließend heiß filtriert. Die Mutterlauge wird eindestilliert. Der Rückstand (83,2 g) wird durch NMR-Spektrum, IR-Spektrum und Elementaranalyse als Dianhydrid

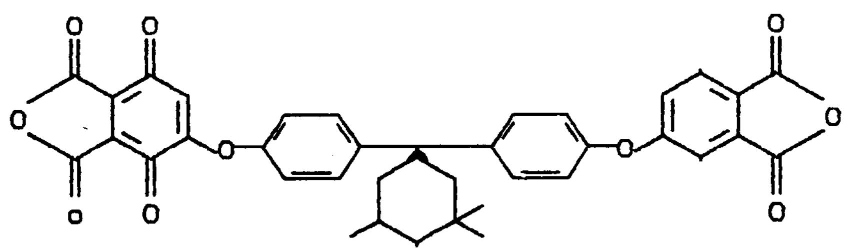

identifiziert.

Elementaranalyse:

| | C | H |
|---|---|---|
| berechnet | 71,5 | 5,45 % |
| gefunden | 73,5 | 5,41 % |

Beispiel 5

44,37 g des Dianhydrids aus Beispiel 4, 8,65 g m-Phenylendiamin und 3,66 mg Natriumhypophosphit-Monohydrat wurden in 96 g o-Dichlorbenzol unter $N_2$ am Wasserabscheider 10 h polykondensiert.

Danach wurde mit 50 ml $CH_2Cl_2$ verdünnt und das Polymer der Struktur

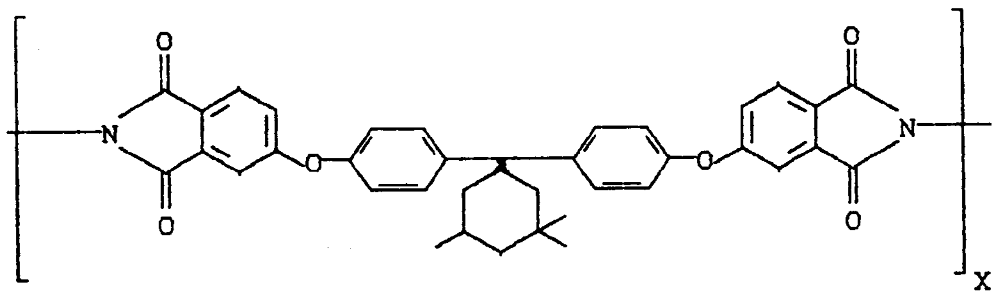

durch Fällen in Methanol isoliert.

**Patentansprüche**

1. Aromatische Etherimide der Formel

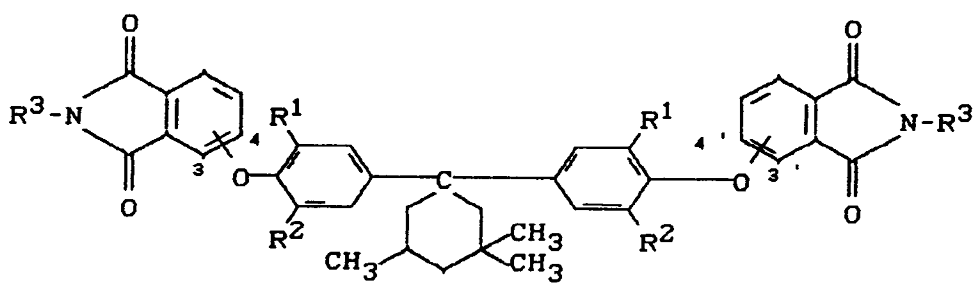

(I)

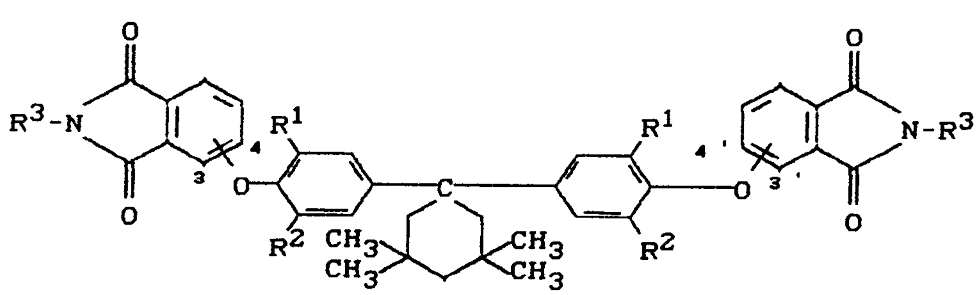

(II) und

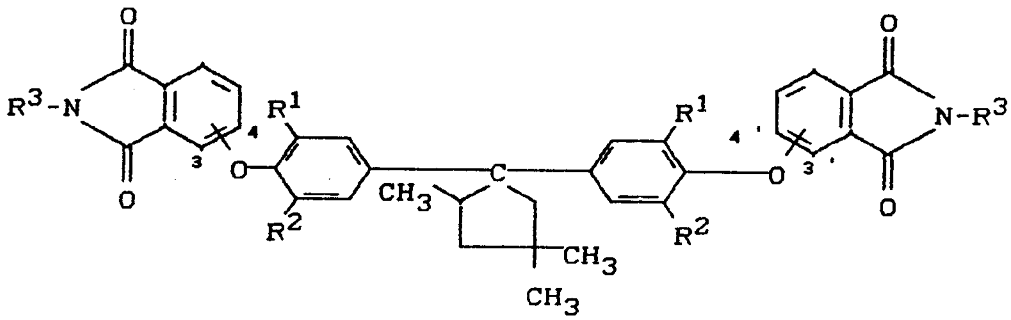

(III),

worin

$R^1$ und $R^2$ unabhängig voneinander Wasserstoff, Halogen, $C_1$-$C_8$-Alkyl, $C_5$-$C_6$-Cycloalkyl, $C_6$-$C_{10}$-Aryl oder $C_7$-$C_{12}$-Aralkyl bedeuten und

$R^3$ für $C_1$-$C_4$-Alkyl, $C_5$-$C_6$-Cycloalkyl oder $C_6$-$C_{10}$-Aryl steht.

**2.** Verfahren zur Herstellung von aromatischen Ethern der Formel

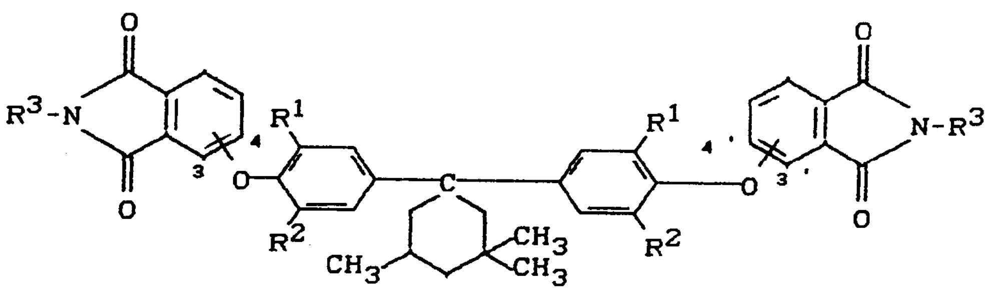

(I)

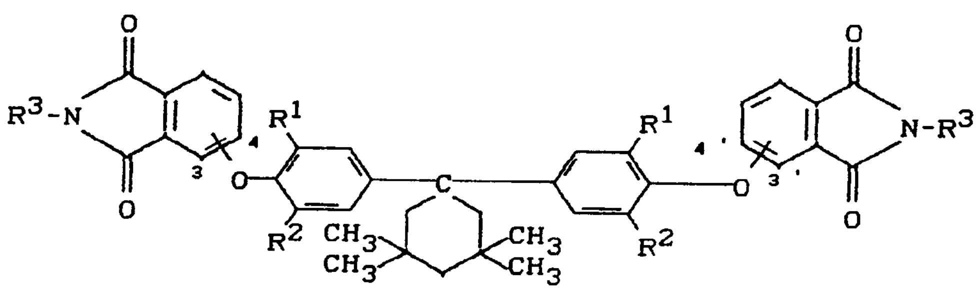

(II) und

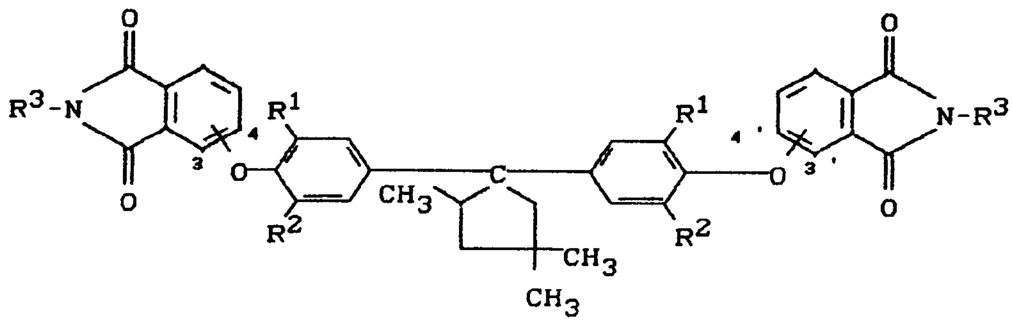

(III),

worin

$R^1$ und $R^2$ unabhängig voneinander Wasserstoff, Halogen, $C_1$-$C_8$-Alkyl, $C_5$-$C_6$-Cycloalkyl, $C_6$-$C_{10}$-Aryl oder $C_7$-$C_{12}$-Aralkyl bedeuten und

$R^3$ für $C_1$-$C_4$-Alkyl, $C_5$-$C_6$-Cycloalkyl oder $C_6$-$C_{10}$-Aryl steht,

wobei man Verbindungen der Formeln

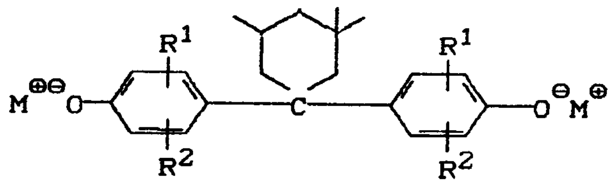

(IV),

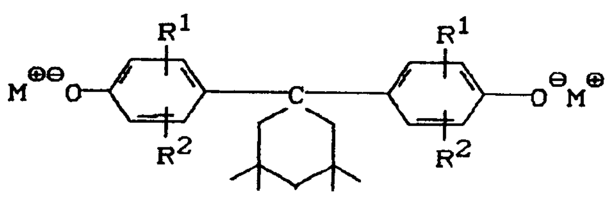

und

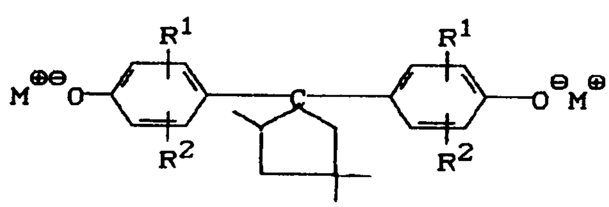

,

worin

$R^1$ und $R^2$ die obengenannte Bedeutung haben und

M für ein Alkalimetall steht,

mit Verbindungen der Formel

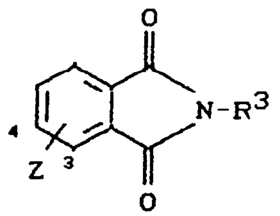

(V),

worin

Z für Fluor, Chlor oder die Nitrogruppe steht, und

$R^3$ die obengenannte Bedeutung besitzt, bei Temperaturen von 20 bis 180°C in Gegenwart eines dipolaren, aprotischen Lösungsmittels umsetzt.

3. Verwendung der aromatischen Etherimide nach Anspruch 1 zur Herstellung von Kunststoffen und Kunststoffmischungen.

## Claims

1. Aromatic ether imides corresponding to the formula

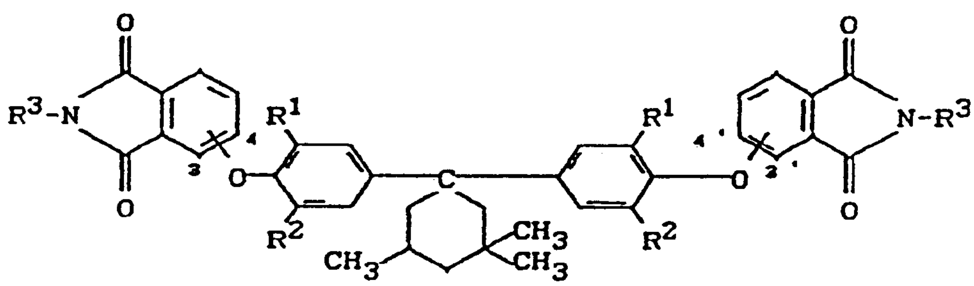

(I)

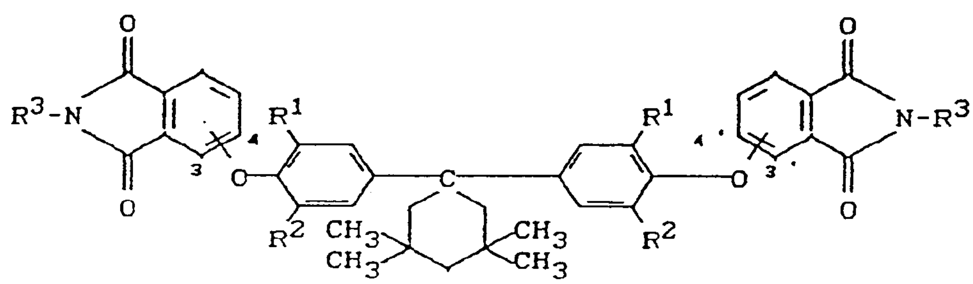

(II)

and

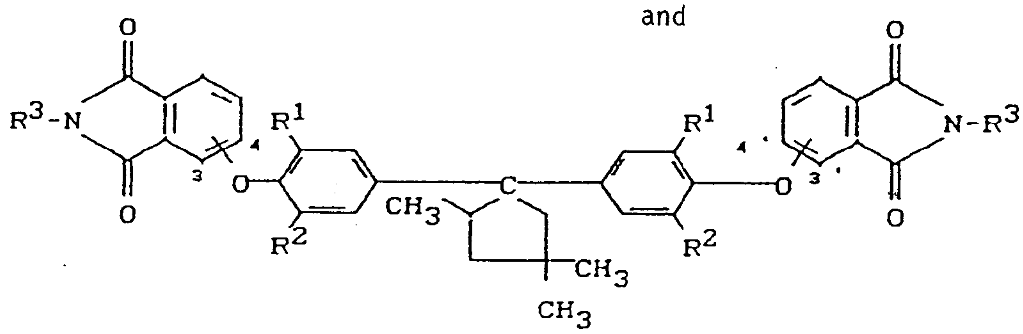

(III),

in which

R¹ and R² independently of one another represent hydrogen, halogen, $C_{1-8}$ alkyl, $C_{5-6}$ cycloalkyl, $C_{6-10}$ aryl or $C_{7-12}$ aralkyl and

R³ represents $C_{1-4}$ alkyl, $C_{5-6}$ cycloalkyl or $C_{6-10}$ aryl.

**2.** A process for the production of aromatic ethers corresponding to the formula

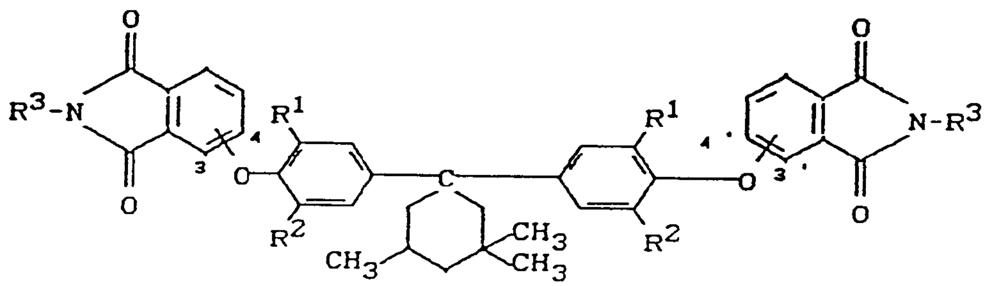

(I)

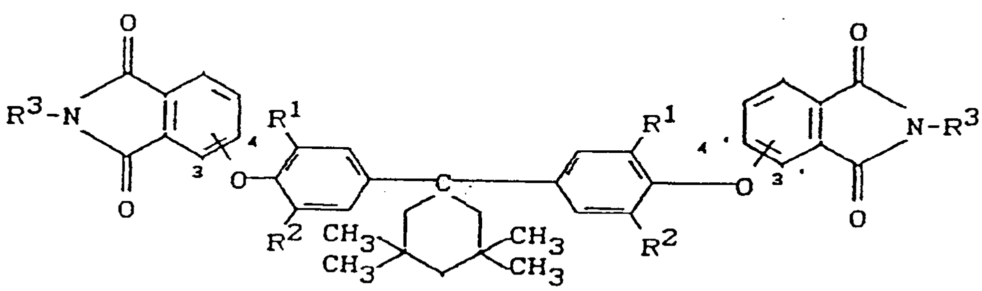

(II)

and

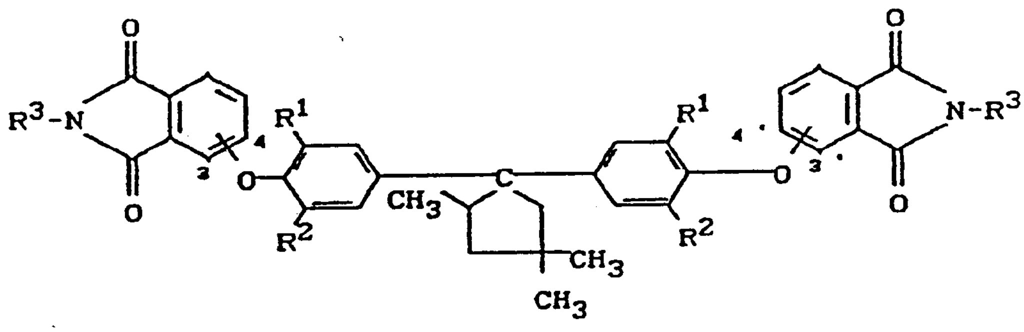

(III),

in Which

$R^1$ and $R^2$ independently of one another represent hydrogen, halogen, $C_{1-8}$ alkyl, $C_{5-6}$ cycloalkyl, $C_{6-10}$ aryl or $C_{7-12}$ aralkyl and

$R^3$ represents $C_{1-4}$ alkyl, $C_{5-6}$ cycloalkyl or $C_{6-10}$ aryl, characterized in that compounds corresponding to the formulae

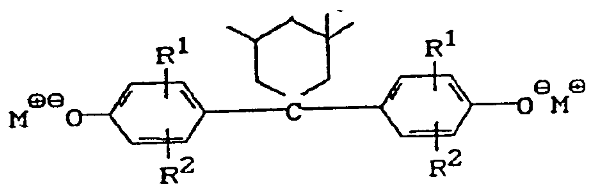

(IV),

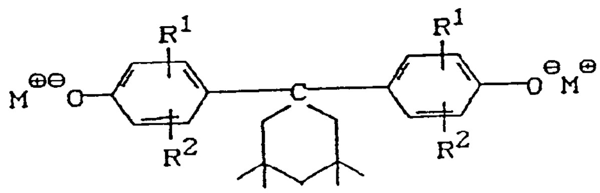

and

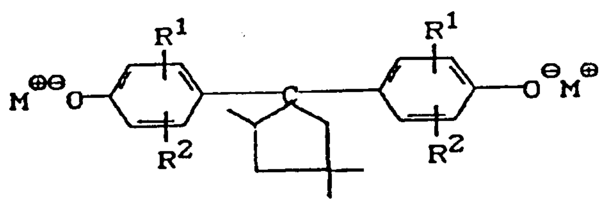

,

in which

$R^1$ and $R^2$ are as defined above and

M is an alkali metal,

are reacted with compounds corresponding to the formula

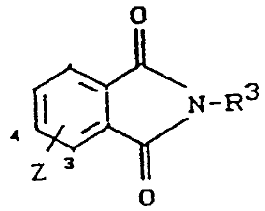

(V),

in which

Z represents fluorine, chlorine or the nitro group and

$R^3$ is as defined above,

at temperatures of 20 to 180°C in the presence of a dipolar aprotic solvent.

3. The use of the aromatic ether imides claimed in claim 1 for the production of polymers and polymer blends.

**Revendications**

1. Etherimides aromatiques de formules

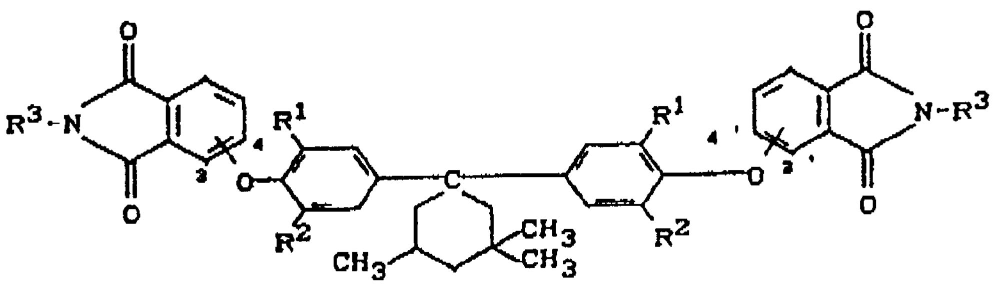

(I)

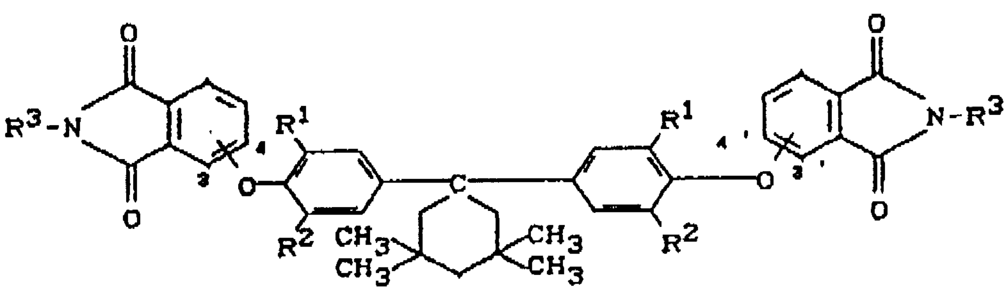

(II) et

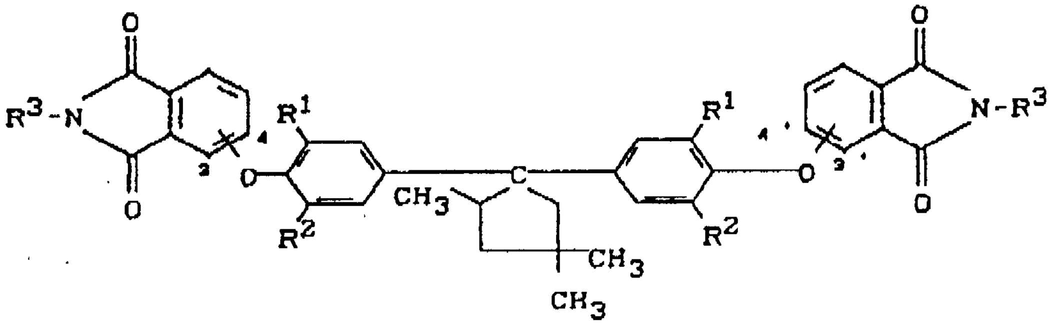

(III),

dans lesquelles

$R^1$ et $R^2$ représentent indépendamment l'un de l'autre l'hydrogène, un halogène, un reste alkyle en $C_1$-$C_8$, cycloalkyle en $C_5$-$C_6$, aryle en $C_6$-$C_{10}$ou aralkyle en $C_7$-$C_{12}$, et

$R^3$ représente un reste alkyle en $C_1$-$C_4$, cycloalkyle en $C_5$-$C_6$ ou aryle en $C_6$-$C_{10}$.

**2.** Procédé pour fabriquer des éthers aromatiques de formules

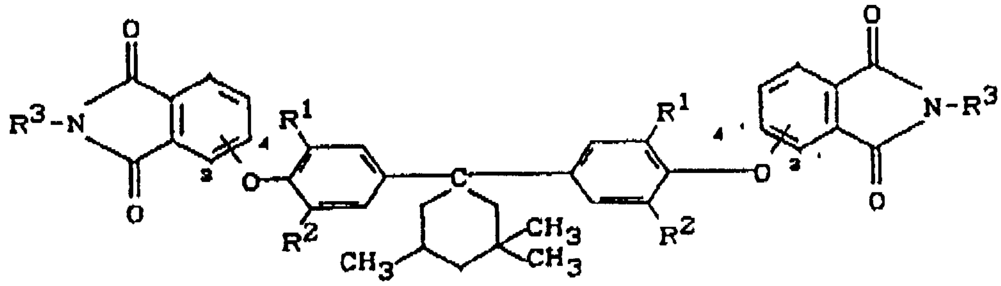

(I)

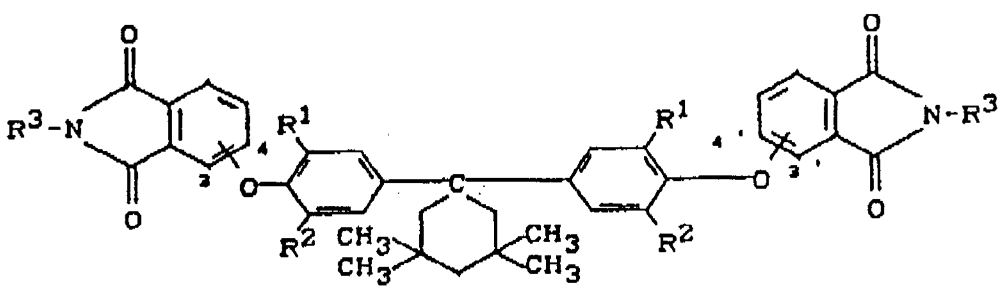

(II) et

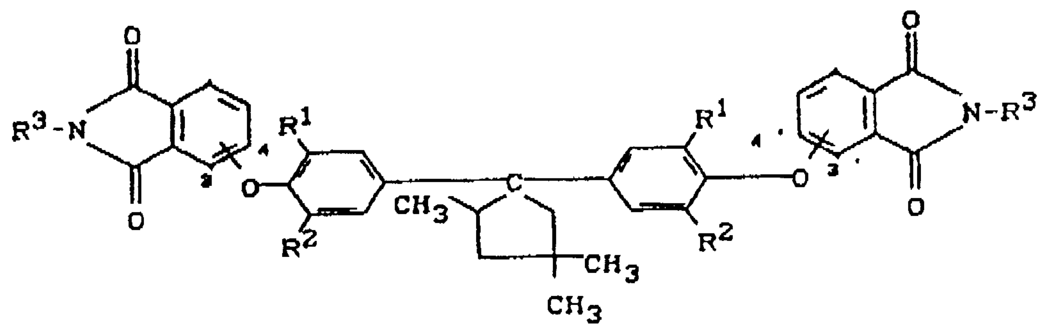

(III),

dans lesquelles

$R^1$ et $R^2$ représentent indépendamment l'un de l'autre l'hydrogène, un halogène, un reste alkyle en $C_1$-$C_8$, cycloalkyle en $C_5$-$C_6$, aryle en $C_6$-$C_{10}$ ou aralkyle en $C_7$-$C_{12}$, et

$R^3$ représente un reste alkyle en $C_1$-$C_4$, cycloalkyle en $C_5$-$C_6$ ou aryle en $C_6$-$C_{10}$,

en faisant réagir des composés répondant aux formules

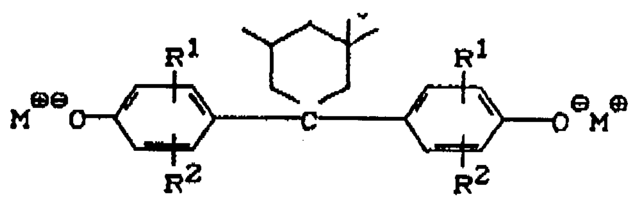

(IV),

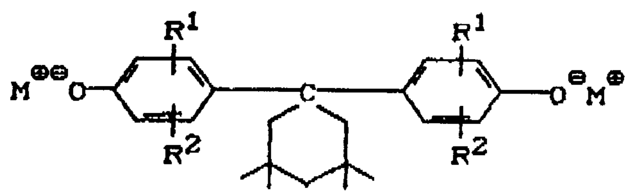

et

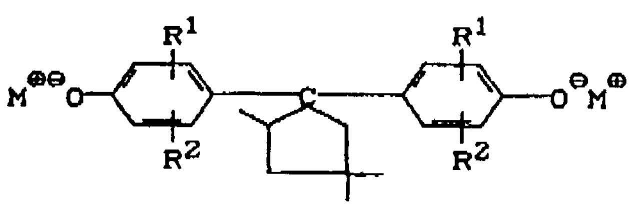

dans lesquelles

$R^1$ et $R^2$ possèdent les significations décrites ci-dessus, et

M représente un métal alcalin,

avec des composés de formule

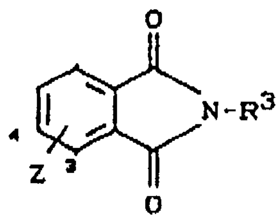

(V),

dans laquelle

Z signifie le fluor, le chlore, un groupe nitro, et

$R^3$ possède la signification décrite ci-dessus,

à des températures de 20 à 180°C, en présence d'un solvant aprotique dipolaire.

3. Utilisation des étherimides aromatiques selon la revendication 1 pour la fabrication de matières synthétiques ou de mélanges de matières synthétiques.